# FASCICULE DE BREVET EUROPEEN

(11) **EP 1 356 839 B1**
(45) Date de publication et mention de la délivrance du brevet: **24.01.2007**
(21) Numéro de dépôt: 03291007.7
(22) Date de dépôt: 24.04.2003
(51) Int. Cl.: A61M 5/50, A61M 5/32

(54) **Ensemble de seringue d'injection de liquide et fourreau pour cet ensemble**
Anordnung einer Flüssigkeitsinjektionsspritze und Hülse für diese Anordnung
Liquid injection syringe assembly and sheeth for this assembly

(30) Priorité: 25.04.2002 FR 0205235
(43) Date de publication de la demande: 29.10.2003
(73) Titulaire: Rexam Pharma La Verpillière, 38290 La Verpilliere Cedex (FR)
(72) Inventeur: Rimlinger, Thierry, 38080 l'Isle d'Abeau (FR); Pouget, Michel, 38300 Domarin (FR); Peysson, Thierry, 69330 Pusignan (FR); Dodier, Philippe, 69110 St Foy Les Lyon (FR)
(74) Mandataire: de la Bigne, Guillaume Michel Marie

(56) Documents cités:
- EP-A- 0 467 173
- WO-A-00/56383
- US-A- 5 342 320

## Description

La présente invention concerne un ensemble de seringue d'injection de liquide et un fourreau pour cet ensemble.

On connaît déjà dans l'état de la technique, notamment d'après US-A-5 562 626, un ensemble de seringue d'injection de liquide, du type comprenant :
- un corps tubulaire de seringue, formant un réservoir pour le liquide, muni d'une extrémité distale portant une aiguille d'injection de liquide,
- un poussoir de seringue monté déplaçable axialement dans le corps entre des positions d'attente et de fin d'injection de liquide, et
- un fourreau tubulaire dans lequel le corps de seringue est monté déplaçable axialement entre une position active, dans laquelle l'aiguille fait saillie à travers une extrémité distale du fourreau, et une position de protection, dans laquelle l'aiguille est escamotée dans le fourreau.

Un ensemble de seringue du type précité est généralement muni de moyens permettant un escamotage automatique de l'aiguille dans le fourreau afin d'éviter qu'une personne manipulant l'ensemble de seringue après son utilisation normale, c'est-à-dire après injection du liquide dans le corps d'un patient, ne se pique accidentellement avec l'aiguille.

Lorsque le corps de seringue est dans sa position de protection par rapport au fourreau, il convient d'éviter tout retour accidentel de l'aiguille d'injection vers sa position saillante à travers le fourreau.

A cet effet, US-A-5 562 626 propose des moyens de solidarisation axiale du corps de seringue et du fourreau activés lorsque le corps de seringue est dans sa position de protection, dans laquelle l'aiguille est escamotée dans le fourreau. Ces moyens de solidarisation comprennent une collerette de blocage venue de matière avec le corps de seringue, si bien que ce corps est spécifique à l'ensemble de seringue décrit dans US-A-5 562 626.

Or, certains produits médicaux sont conditionnés directement dans des corps de seringue standard (seringue pré-remplie), généralement en verre, obturés par des pistons auxquels seront reliés par la suite des poussoirs d'actionnement des pistons.

L'invention a pour but de proposer un ensemble de seringue du type précité susceptible d'être équipé d'un corps de seringue relativement standard, fabriqué par exemple en verre, cet ensemble étant équipé de moyens empêchant efficacement, après utilisation de la seringue, tout retour accidentel de l'aiguille d'injection vers sa position saillante à travers le fourreau.

A cet effet, l'invention a pour objet un ensemble de seringue d'injection de liquide, du type précité, **caractérisé en ce qu'il** comprend des moyens, portés par le poussoir et le fourreau, de solidarisation axiale du poussoir et du fourreau dans une position relative de ce poussoir et de ce fourreau autorisant, d'une part, le positionnement du corps dans sa position de protection relativement au fourreau et, d'autre part, le positionnement du poussoir dans sa position de fin d'injection relativement au corps.

Suivant d'autres caractéristiques de différents modes de réalisation de cet ensemble de seringue :
- l'ensemble comprend :
   ■ des moyens de rappel élastique du corps vers sa position de protection,
   ■ des moyens de retenue du corps en position active relativement au fourreau, s'opposant à la force élastique des moyens de rappel,
   ■ un organe, porté par le poussoir, destiné à coopérer avec les moyens de retenue pour les désactiver lorsque l'ensemble est dans une configuration de fin d'injection,
   les moyens de solidarisation axiale du poussoir et du fourreau comportant des butées axiales opposées, dites butées de solidarisation, entre lesquelles l'organe de désactivation des moyens de retenue est destiné à s'encliqueter ;
- en position encliquetée entre les butées de solidarisation, l'organe de désactivation des moyens de retenue est susceptible de coopérer avec chacune de ces butées de solidarisation ;
- les butées de solidarisation sont délimitées par des pattes axiales, ménagées dans le fourreau, escamotables par déformation élastique radiale ;
- l'organe de libération des moyens de retenue est formé par une collerette venue de matière avec le poussoir, dite collerette de poussoir ;
- le corps de seringue est muni d'une extrémité proximale comportant une collerette, dite collerette de corps, les moyens de retenue comprenant des butées axiales opposées, dites butées de retenue, entre lesquelles est retenue la collerette de corps, ces butées de retenue étant délimitées par un épaulement interne du fourreau, dit épaulement de retenue, et au moins une patte axiale, ménagée dans ce fourreau, escamotable par déformation radiale ;
- le poussoir étant dans sa position de fin d'injection relativement au corps, la collerette de corps est destinée à s'encliqueter entre les butées de solidarisation, de façon que la collerette de corps est susceptible de coopérer avec la butée distale de solidarisation et l'organe de désactivation des moyens de retenue est susceptible de coopérer avec la butée proximale de solidarisation ;
- des moyens de rappel du corps de seringue vers sa position de protection comprennent un ressort de poussée en appui, d'une part, sur un épaulement interne du fourreau, dit épaulement de rappel, intercalé entre l'extrémité distale du fourreau et l'épaulement de retenue, et d'autre part, sur la collerette de corps de seringue ;
- le fourreau porte des moyens de préhension externes destinés à être saisis par les doigts d'un utilisateur pour injecter le liquide par rapprochement axial d'une extrémité proximale du poussoir et des moyens de préhension ;
- les moyens de préhension comprennent un épaulement externe du fourreau.

L'invention a également pour objet un fourreau pour ensemble de seringue d'injection de liquide, **caractérisé en ce qu'**il est destiné à un ensemble tel que défini ci-dessus.

L'invention sera mieux comprise à la lecture de la description qui va suivre, donnée uniquement à titre d'exemple et faite en se référant aux dessins dans lesquels :
- les figures 1 à 3 sont des vues en coupe axiale d'un ensemble de seringue selon un premier mode de réalisation de l'invention dans trois configurations d'utilisation de cet ensemble ; et
- les figures 4 à 6 sont des vues similaires au figures précédentes d'un ensemble de seringue selon un second mode de réalisation de l'invention.

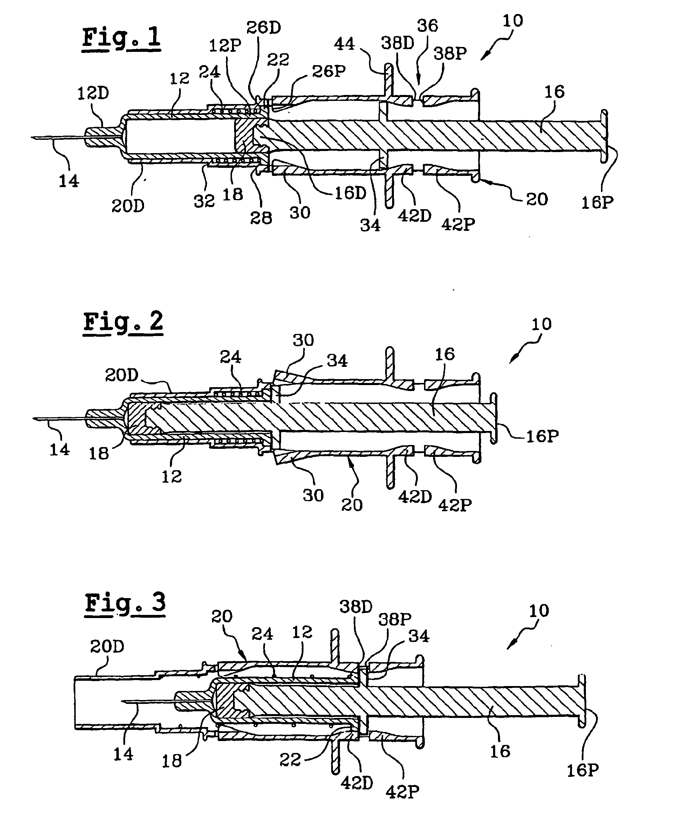

On a représenté sur les figures 1 à 3 un ensemble de seringue d'injection de liquide, notamment un liquide médical, selon un premier mode de réalisation de l'invention. Cet ensemble de seringue est désigné par la référence générale 10.

L'ensemble de seringue 10 comprend un corps tubulaire 12 de seringue, de type classique, formant un réservoir pour le liquide. Ce corps 12 porte une aiguille 14 d'injection du liquide.

L'ensemble de seringue 10 comprend également un poussoir 16 monté déplaçable axialement dans le corps 12 entre une position d'attente, telle que représentée sur la figure 1, et une position de fin d'injection de liquide, telle que représentée sur la figure 2.

Dans ce qui suit un élément sera qualifié de proximal ou de distal selon qu'il est proche ou éloigné axialement de l'extrémité du poussoir 16 destiné à être actionné par un utilisateur.

Le poussoir 16, fabriqué par exemple en plastique, comporte une extrémité proximale 16P, externe au corps 12, et une extrémité distale 16D, interne au corps 12, portant un piston classique 18. L'extrémité proximale 16P forme une extrémité de manoeuvre du poussoir 16.

L'ensemble de seringue 10 comporte un fourreau de sécurité tubulaire 20 dans lequel est logé le corps 12 de seringue. Le fourreau 20, de révolution, est fabriqué par exemple en matière plastique ayant le cas échéant une transparence suffisante pour permettre la visualisation du liquide contenu dans le corps 12 de seringue ou d'inscriptions portées par ce corps 12.

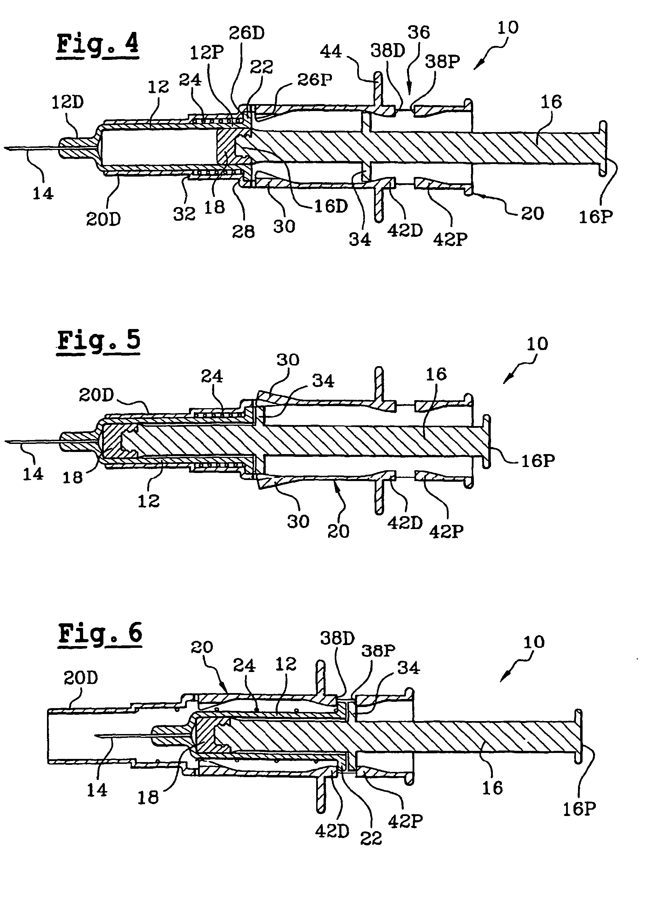

Le corps 12 de seringue est monté déplaçable axialement dans le fourreau 20 entre une position active, dans laquelle l'aiguille 14 fait saillie à travers une extrémité distale 20D du fourreau, et une position de protection, dans laquelle l'aiguille 14 est escamotée dans le fourreau 20. La position active est représentée sur les figures 1 et 2. La position de protection est représentée sur la figure 3.

On notera que le corps 12 de seringue comporte une extrémité distale 12D portant l'aiguille 14 et une extrémité proximale 12P munie d'une collerette, dite collerette 22 de corps.

L'ensemble 10 de seringue comprend des moyens de retenue du corps 12 en position active relativement au fourreau 20. Ces moyens de retenue s'opposent à la force élastique de moyens de rappel comprenant un ressort de poussée 24.

Dans l'exemple illustré, les moyens de retenue comprennent des butées axiales opposées, dites butées distale 26D et proximale 26P de retenue. La butée distale de retenue 26D est délimitée par un épaulement interne du fourreau 20, dit épaulement 28 de retenue. La butée proximale de retenue 26P est délimitée par au moins une patte axiale 30, ménagée dans le fourreau 20, escamotable par déformation radiale, par exemple par déformation élastique radiale.

De préférence, le fourreau 20 comporte deux pattes axiales de retenue 30, diamétralement opposées.

Lorsque l'ensemble de seringue 10 est dans une configuration d'attente, la collerette 22 de corps est retenue axialement entre les butées de retenue 26D, 26P, comme cela est représenté sur la figure 1.

Le ressort 24 est en appui, d'une part, sur un épaulement interne du fourreau 20, dit épaulement 32 de rappel, et d'autre part, sur la collerette 22 de corps. On notera que l'épaulement de rappel 32 est intercalé entre l'extrémité distale 20D du fourreau et l'épaulement de retenue 28.

Les moyens de retenue du corps 12 en position active sont destinés à être désactivés à l'aide d'un organe porté par le poussoir 16 par exemple une collerette venue de matière avec ce poussoir 16, dite collerette 34 de poussoir.

Lorsque l'ensemble de seringue 10 est dans une configuration de fin d'injection de liquide, comme cela est représenté sur la figure 2, la collerette 34 de poussoir coopère avec des rampes ménagées sur les pattes axiales 30 pour déformer ces dernières. Ceci a pour effet d'écarter radialement les pattes 30 de la collerette 22 de corps et ainsi de libérer les moyens de retenue du corps 12 en position active.

Une fois les moyens de retenue libérés, le ressort 24 rappelle élastiquement le corps 12 de seringue vers sa position de protection représentée sur la figure 3.

L'ensemble de seringue 10 comprend des moyens 36 de solidarisation axiale du poussoir 16 et du fourreau 20 dans une position relative qui est représentée sur la figure 3. Cette position relative du poussoir 16 et du fourreau 20 autorise, d'une part, le positionnement du corps 12 dans sa position de protection relativement au fourreau 20 et, d'autre part, le positionnement du poussoir 16 dans sa position de fin d'injection relativement au corps 12.

Dans l'exemple représenté, les moyens de solidarisation axiale 36 comportent des butées axiales opposées dites butées distale 38D et proximale 38P de solidarisation, entre lesquelles la collerette 34 de poussoir est destinée à s'encliqueter.

Les butées de solidarisation 38D, 38P sont délimitées par des pattes axiales distale 42D et proximale 42P, ménagées dans le fourreau 20, escamotables par déformation élastique radiale.

De préférence, le fourreau 20 comporte deux paires de pattes axiales 42D, 42P, diamétralement opposées, chaque paire comportant une patte distale 42D et une patte proximale 42P.

Le fourreau 20 porte des moyens externes de préhension destinés à être saisis par les doigts d'un utilisateur pour injecter le liquide par rapprochement axial de l'extrémité de manoeuvre 16P du poussoir et de ces moyens de préhension.

Dans l'exemple décrit, les moyens de préhension comprennent deux pattes radiales externes 44 solidaires du fourreau 20.

Avant utilisation, l'ensemble de seringue 10 est dans la configuration d'attente telle que représentée sur la figure 1. Le cas échéant, un capuchon amovible de protection de l'aiguille 14 (non représenté sur la figure) est emboîté sur l'extrémité distale 12D du corps de seringue.

Dans cette configuration, la collerette 34 de poussoir est intercalée axialement entre les pattes de retenue 30 et les pattes distales 42D de solidarisation.

Pour réaliser l'injection du liquide, l'utilisateur saisit l'ensemble de seringue 10 de façon à enfoncer le poussoir 16 dans le corps 12 de seringue en rapprochant axialement l'extrémité 16P du poussoir des pattes de préhension 44.

L'utilisateur enfonce le poussoir 16 jusqu'à atteindre la position de fin d'injection représentée sur la figure 2.

Dans la configuration de l'ensemble de seringue 10 représentée sur la figure 2, les pattes axiales 30 de retenue du corps 12 sont écartées radialement de la collerette 22 de corps par la collerette 34 de poussoir.

Du fait de l'escamotage des butées de retenue 26P portées par les pattes 30 et du relâchement de la pression exercée par l'utilisateur sur l'extrémité de manoeuvre 16P du poussoir, le ressort 24 se détend et pousse le corps 12 de seringue vide vers sa position de protection telle que représentée sur la figure 3.

Au cours du déplacement du corps 12 de seringue vers sa position de protection relativement au fourreau 20, la collerette 34 de poussoir coopère, tout d'abord, avec les pattes distales 42D de solidarisation pour les escamoter radialement puis s'encliquette entre les butées de solidarisation 38D, 38P, comme cela est représenté sur la figure 3.

En position encliquetée entre les butées de solidarisation 38D, 38P, la collerette 34 de poussoir est susceptible de coopérer avec chacune de ces butées de solidarisation 38D, 38P.

Ainsi, dans la configuration de l'ensemble de seringue 10 représentée sur la figure 3, l'aiguille 14 est escamotée dans le fourreau 20. Par ailleurs, le poussoir 16 est immobilisé axialement par rapport au fourreau 20 si bien que le corps 12 ne peut plus être déplacé vers sa position active par actionnement involontaire du poussoir 16.

Toute séparation intempestive de la collerette 34 d'avec les pattes 42D, 42P pourra être empêchée par un dimensionnement approprié de ces pattes 42D, 42P, notamment en limitant le jeu entre la collerette 34 et les butées 38D, 38P.

On notera que les pattes proximales 42P sont munies de rampes, facilitant, lors du montage initial de l'ensemble de seringue 10, l'introduction du poussoir 16 dans le fourreau 20 en vue de son accrochage sur le piston 18 bouchant le corps 12 de seringue préalablement logé dans le fourreau 20.

Parmi les avantages de l'invention, on notera que le corps de l'ensemble de seringue selon l'invention a une forme simple bien adaptée, notamment, à une fabrication standardisée en verre ou en plastique et à une utilisation dans une seringue pré-remplie.

La structure de l'ensemble de seringue selon l'invention est très simple. En effet, il suffit d'équiper une seringue du poussoir, 16, du fourreau 20, réalisé en une seule pièce, et du ressort 24 pour obtenir l'ensemble selon l'invention.

Le fonctionnement de l'ensemble de seringue selon l'invention est tel qu'il requiert de la part de l'utilisateur les mêmes gestes que pour une seringue classique ne comportant pas de moyens d'escamotage de l'aiguille.

Les moyens de retenue du corps de seringue en position active sont désactivés automatiquement dès la fin de l'injection de liquide. Les moyens de solidarisation axiale du poussoir avec le fourreau sont activés automatiquement lorsque l'utilisateur relâche la pression exercée sur le poussoir.

La désactivation des moyens de retenue et l'activation des moyens de solidarisation ci-dessus se font donc automatiquement alors que l'utilisateur manipule l'ensemble de seringue d'une seule main en réalisant un geste tout à fait classique.

Par ailleurs, les moyens de solidarisation axiale du poussoir 16 et du fourreau 20 empêchent efficacement, après utilisation de la seringue, tout retour accidentel de l'aiguille d'injection vers sa position saillante à travers le fourreau.

On a représenté sur les figures 4 à 6 un ensemble de seringue d'injection de liquide selon un second mode de réalisation de l'invention. Sur ces figures, les éléments analogues à ceux des figures 1 à 3 sont désignés par des références identiques.

Avant utilisation, l'ensemble de seringue 10 est dans la configuration d'attente telle que représentée sur la figure 4.

Comme dans le premier mode de réalisation de l'invention, pour réaliser l'injection du liquide, l'utilisateur saisit l'ensemble de seringue 10 de façon à enfoncer le poussoir 16 dans le corps 12 de seringue en rapprochant axialement l'extrémité 16P du poussoir des pattes de préhension 44.

L'utilisateur enfonce le poussoir 16 jusqu'à atteindre la position de fin d'injection représentée sur la figure 5. Dans cette configuration, les pattes axiales 30 de retenue du corps 12 sont écartées radialement de la collerette 22 de corps par la collerette 34 de poussoir.

La collerette 22 de corps, poussée par le ressort 24, est destinée à s'encliqueter entre les butées de solidarisation 38D, 38P, comme cela est représenté sur la figure 6. Dans cette configuration, la collerette 22 de corps est susceptible de coopérer avec la butée distale de solidarisation 38D et la collerette 34 de poussoir est susceptible de coopérer avec la butée proximale de solidarisation 38P.

Ainsi, dans la configuration de l'ensemble de seringue 10 représentée sur la figure 6 (aiguille 14 escamotée dans le fourreau 20), le poussoir 16 et le corps 12 sont immobilisés axialement par rapport au fourreau 20, si bien que ce corps 12 ne peut plus être déplacé vers sa position active.

L'invention ne se limite pas aux modes de réalisation décrits ci-dessus.

En particulier, l'aiguille 14 peut être rapportée sur le corps 12 à l'aide de moyens d'assemblage manuel tels que des moyens de vissage ou d'encliquetage.

Par ailleurs, les moyens 26D, 26P de retenue du corps 12 en position active relativement au fourreau 20 peuvent être désactivés avant même que le poussoir 16 atteigne la position de fin d'injection. Il suffit pour cela d'adapter en conséquence les formes ou positions des butées de retenue 26P portées par les pattes 30 et de la collerette 34 de poussoir.

Un tel agencement permet de provoquer l'escamotage de l'aiguille 14, et donc de protéger l'utilisateur, même si ce dernier n'a pas injecté la totalité de liquide contenu dans le corps de seringue.

## Revendications

1. Ensemble de seringue d'injection de liquide, du type comprenant :
- un corps (12) tubulaire de seringue, formant un réservoir pour le liquide, muni d'une extrémité distale (12D) portant une aiguille (14) d'injection de liquide,
- un poussoir (16) de seringue monté déplaçable axialement dans le corps (12) entre des positions d'attente et de fin d'injection de liquide, et
- un fourreau (20) tubulaire dans lequel le corps (12) de seringue est monté déplaçable axialement entre une position active, dans laquelle l'aiguille (14) fait saillie à travers une extrémité distale (20D) du fourreau (20), et une position de protection, dans laquelle l'aiguille (14) est escamotée dans le fourreau (20), portés par le poussoir (16) et le fourreau (20),
**caractérisé en ce qu'**il comprend des moyens (34, 38D, 38P) de solidarisation axiale du poussoir (16) et du fourreau (20) dans une position relative de ce poussoir (16) et de ce fourreau (20) autorisant, d'une part, le positionnement du corps (12) dans sa position de protection relativement au fourreau (20) et, d'autre part, le positionnement du poussoir (16) dans sa position de fin d'injection relativement au corps (12).

2. Ensemble de seringue selon la revendication 1, **caractérisé en ce qu'**il comprend :
- des moyens (24) de rappel élastique du corps (12) vers sa position de protection,
- des moyens (26D, 26P, 30) de retenue du corps (12) en position active relativement au fourreau (20), s'opposant à la force élastique des moyens de rappel (24),
- un organe (34), porté par le poussoir (16), destiné à coopérer avec les moyens de retenue (30) pour les désactiver lorsque l'ensemble (10) est dans une configuration de fin d'injection,
les moyens de solidarisation axiale du poussoir (16) et du fourreau (20) comportant des butées axiales opposées, dites butées distale(38) et proximale (38P) de solidarisation, entre lesquelles l'organe (34) de désactivation des moyens de retenue est destiné à s'encliqueter.

3. Ensemble de seringue selon la revendication 2, **caractérisé en ce que**, en position encliquetée entre les butées de solidarisation (38D, 38P), l'organe (34) de désactivation des moyens de retenue est susceptible de coopérer avec chacune de ces butées de solidarisation (38D, 38P).

4. Ensemble de seringue selon la revendication 2 ou 3, **caractérisé en ce que** les butées (38D, 38P) de solidarisation sont délimitées par des pattes axiales (42D, 42P), ménagées dans le fourreau (20), escamotables par déformation élastique radiale.

5. Ensemble de seringue selon l'une quelconque des revendications 2 à 4, **caractérisé en ce que** l'organe de libération des moyens de retenue est formé par une collerette (34) venue de matière avec le poussoir (16), dite collerette de poussoir (16).

6. Ensemble de seringue selon l'une quelconque des revendications 2 à 5, **caractérisé en ce que** le corps (12) de seringue est muni d'une extrémité proximale (12P) comportant une collerette, dite collerette (22) de corps (12), les moyens de retenue comprenant des butées axiales opposées (26D, 26P), dites butées de retenue, entre lesquelles est retenue la collerette (22) de corps (12), ces butées de retenue (26D, 26P) étant délimitées par un épaulement interne du fourreau (20), dit épaulement de retenue (28), et au moins une patte axiale (30), ménagée dans ce fourreau (20) escamotable par déformation radiale.

7. Ensemble de seringue selon la revendication 6, **caractérisé en ce que**, le poussoir (16) étant dans sa position de fin d'injection relativement au corps (12), la collerette (22) de corps (12) est destinée à s'encliqueter entre les butées de solidarisation (38D, 38P), de façon que la collerette (22) de corps (12) est susceptible de coopérer avec la butée distale (38D) de solidarisation et l'organe (34) de désactivation des moyens de retenue est susceptible de coopérer avec la butée proximale (38P) de solidarisation.

8. Ensemble de seringue selon la revendication 6 ou 7, **caractérisé en ce que** des moyens de rappel du corps (12) de seringue vers sa position de protection comprennent un ressort de poussée (24) en appui, d'une part, sur un épaulement interne du fourreau (20), dit épaulement (32) de rappel, intercalé entre l'extrémité distale (20D) du fourreau (20) et l'épaulement de retenue (28), et d'autre part, sur la collerette (22) de corps (12) de seringue.

9. Ensemble de seringue selon l'une quelconque des revendications précédentes, **caractérisé en ce que** le fourreau (20) porte des moyens de préhension externes (44) destinés à être saisis par les doigts d'un utilisateur pour injecter le liquide par rapprochement axial d'une extrémité proximale (16P) du poussoir (16) et des moyens de préhension (44).

10. Ensemble de seringue selon la revendication 9, **caractérisé en ce que** les moyens de préhension comprennent un épaulement externe (44) du fourreau (20).

11. Fourreau (20) pour ensemble de seringue d'injection de liquide selon l'une quelconque des revendications précédentes, dans lequel le corps (12) de seringue est destiné à être monté déplaçable axialement entre une position active, dans laquelle l'aiguille (14) fait saillie à travers une extrémité distale (20D) du fourreau (20), et une position de protection, dans laquelle l'aiguille (14) est escamotée dans le fourreau (20), **caractérisé en ce qu'**il comprend des moyens (38D, 38P) destinés à coopérer avec des moyens complémentaires (34) du poussoir (16) pour solidariser axialement le poussoir (16) et le fourreau (20) dans une position relative de ce poussoir (16) et de ce fourreau (20) autorisant, d'une part, le positionnement du corps (12) dans sa position de protection relativement au fourreau (20) et, d'autre part, le positionnement du poussoir (16) dans sa position de fin d'injection relativement au corps (12).

## Claims

1. A liquid-injection syringe assembly of the type comprising:
- a tubular syringe body (12) forming a reservoir for the liquid, the body being provided with a distal end (12D) carrying a liquid-injection needle (14) ;
- a syringe plunger (16) mounted to be axially movable in the body (12) between a ready position and an end-of-injection position; and
- a tubular sheath (20) in which the syringe body (12) is mounted to be axially movable between an active position in which the needle (14) projects from the distal end (20D) of the sheath (20), and a protecting position in which the needle (14) is retracted inside the sheath (20) ;
**characterized in that** the assembly includes axial securing means (34, 38D, 38P) carried by the plunger (16) and the sheath (20) for securing the plunger (16) and the sheath (20) axially in a relative position of said plunger (16) and said sheath (20) serving both to position the body (12) in its protecting position relative to the sheath (20), and to position the plunger (16) in its end-of-injection (20) position relative to the body (12).

2. A syringe assembly according to claim 1, comprising:
- return means (24) for resiliently returning the body (12) towards its protecting position;
- retaining means (26D, 26P, 30) for retaining the body (12) in its active position relative to the sheath (20), opposing the resilient force of the return means (24) ; and
- a member (34) carried by the plunger (16) for cooperating with the retaining means (30) to deactivate them when the assembly (10) is in an end-of-injection configuration;
the axial securing means (16) for securing the plunger (16) and the sheath (20) comprising axially opposing distal (38) and proximal (38) securing abutments between which the member (34) for deactivating the retaining means is designed to snap-fasten.

3. A syringe assembly according to claim 2, wherein, in its position snap-fastened between the securing abutments (38D, 38P), the member (34) for deactivating the retaining means can co-operate with each of said securing abutments (34D, 38P).

4. A syringe assembly according to claim 2 or 3, wherein the securing abutments (38D, 38P) are defined by axial tabs (42D, 42P) formed in the sheath (20) and retractable by radial elastic deformation.

5. A syringe assembly according to any of the claims 2 to 4, wherein the member for releasing the retaining means is formed by a flange (34) integrally molded with the plunger and referred to as the plunger (16) flange.

6. A syringe assembly according to any of the claims 2 to 5, wherein the syringe body (12) is provided with a proximal end (12P) having a flange (22) referred to as the body flange, the retaining means comprising axially opposing retaining abutments (26D, 26P) between which the body flange (22) is retained, said retaining abutments (26D, 26P) being defined by an internal retaining shoulder (28) of the sheath (20) and by at least one axial tab (30) formed inside said sheath (20) and retractable by radial deformation.

7. A syringe assembly according to claim 6, wherein, when the plunger (16) is in its end-of-injection position relative to the body (12), the body flange (22) is designed to become snap-fastened between the securing abutments (38D, 38P) in such a manner that the body flange (22) can co-operate with the distal securing abutment (38D) and the member (34) for deactivating the retaining means can co-operate with the proximal securing abutment (38P).

8. A syringe assembly according to claim 6 or 7, wherein the return means for returning the syringe body (12) towards its protecting position comprise a thrust spring (24) bearing firstly against an internal "return" shoulder (32) of the sheath (20) interposed between the distal end (20D) of the sheath and the retaining shoulder (28), and also against the flange (22) of the syringe body (12).

9. A syringe assembly according to any of the claims 1 to 8, wherein the sheath (20) carries external grip means (44) for being held by the fingers of a user to inject the liquid by moving the proximal end (16P) of the plunger (16) axially towards the grip means (44).

10. A syringe assembly according to claim 9, wherein the grip means comprise an external shoulder (44) on the sheath (20).

11. A sheath (20) for a liquid-injection syringe assembly according to any of claims 1 to 10, in which the syringe body (12) is designed to be mounted so as to be axially movable between an active position in which the needle (14) projects from a distal end (20D) of the sheath (20), and a protecting position in which the needle (14) is retracted inside the sheath (20), the sheath (20) including means (38D, 38P) designed to co-operate with complementary means of the plunger (16) to secure the plunger (16) and the sheath (20) axially in a relative position of said plunger (16) and said sheath (20) serving firstly to position the body (12) in its protecting position relative to the sheath (20), and secondly to position the plunger (16) in its end-of-injection position relative to the body (12).

## Patentansprüche

1. Anordnung einer Flüssigkeitsinjektionsspritze vom Typ umfassend:
- einen röhrenförmigen Spritzenkörper (12), welcher einen Behälter für die Flüssigkeit bildet und mit einem distalen Ende (12D) versehen ist, das eine Flüssigkeitsinjektionsnadel (14) trägt,
- einen Spritzenschieber (16), der im Körper (12) zwischen einer Warteposition und einer Endposition der Flüssigkeitsinjektion axial beweglich montiert ist, und
- eine röhrenförmige Hülse (20), in der der Spritzenkörper (12) zwischen einer aktiven Position, in der die Nadel (14) über ein distales Ende (20D) der Hülse (20) herausragt, und einer Schutzposition, in der die Nadel (14) in die Hülse (20) zurückgezogen ist, axial beweglich montiert ist,
**dadurch gekennzeichnet, dass** sie durch den Schieber (16) und die Hülse (20) getragene Mittel (34, 38D, 38P) zur axialen festen Verbindung des Schiebers (16) und der Hülse (20) in einer relativen Position dieses Schiebers (16) und dieser Hülse (20) umfasst, durch die einerseits die Positionierung des Körpers (12) in seiner Schutzposition relativ zur Hülse (20) und andererseits die Positionierung des Schiebers (16) in seiner Injektionsendposition relativ zum Körper (12) ermöglicht werden.

2. Spritzenanordnung nach Anspruch 1, **dadurch gekennzeichnet, dass** sie Folgendes umfasst:
- Mittel (24) zum elastischen Zurückziehen des Körpers (12) in Richtung seiner Schutzposition,
- Mittel (26D, 26P, 30) zum Zurückhalten des Körpers (12) in der aktiven Position relativ zur Hülse (20), die sich der elastischen Kraft der Rückzugsmittel (24) widersetzen,
- ein durch den Schieber (16) getragenes Element (34), das dazu bestimmt ist, mit den Zurückhaltemitteln (30) zusammenzuwirken, um sie zu deaktivieren, wenn die Anordnung (10) sich in einer Injektionsendkonfiguration befindet,
wobei die Mittel zur axialen festen Verbindung des Schiebers (16) und der Hülse (20) gegenüberliegende axiale Anschläge, genannt distaler (38) und proximaler Anschlag (38P) zur festen Verbindung, umfassen, zwischen denen das Element (34) zur Deaktivierung der Zurückhaltemittel dazu bestimmt ist einzurasten.

3. Spritzenanordnung nach Anspruch 2, **dadurch gekennzeichnet, dass** das Element (34) zur Deaktivierung der Rückhaltemittel in der zwischen den Anschlägen zur festen Verbindung (38D, 38P) eingerasteten Position in der Lage ist, mit jedem dieser Anschläge zur festen Verbindung (38D, 38P) zusammenzuwirken.

4. Spritzenanordnung nach Anspruch 2 oder 3, **dadurch gekennzeichnet, dass** die Anschläge (38D, 38P) zur festen Verbindung durch axiale Haken (42D, 42P) begrenzt werden, welche in die Hülse (20) eingelassen und durch elastische radiale Verformung versenkbar sind.

5. Spritzenanordnung nach einem der Ansprüche 2 bis 4, **dadurch gekennzeichnet, dass** das Element zur Freisetzung der Rückhaltemittel durch einen Bund (34), bestehend aus einem Stück mit dem Schieber (16), genannt Schieberbund (16), gebildet wird.

6. Spritzenanordnung nach einem der Ansprüche 2 bis 5, **dadurch gekennzeichnet, dass** der Spritzenkörper (12) mit einem proximalen Ende (12P) versehen ist, das einen Bund, genannt Bund (22) des Körpers (12), umfasst, wobei die Rückhaltemittel gegenüberliegende axiale Anschläge (26D, 26P), genannt Rückhalteanschläge, umfassen, zwischen denen der Bund (22) des Körpers (12) zurückgehalten wird, wobei diese Rückhalteanschläge (26D, 26P) durch einen Innenansatz der Hülse (20), genannt Rückhalteansatz (28), und mindestens einen axialen Haken (30), welcher in diese Hülse (20) eingelassen und durch radiale Verformung versenkbar ist, begrenzt werden.

7. Spritzenanordnung nach Anspruch 6, **dadurch gekennzeichnet, dass**, wenn sich der Schieber (16) in seiner Injektionsendposition relativ zum Körper (12) befindet, der Bund (22) des Körpers (12) dazu bestimmt ist, zwischen den Anschlägen zur festen Verbindung (38D, 38P) einzurasten, sodass der Bund (22) des Körpers (12) in der Lage ist, mit dem distalen Anschlag (38D) zur festen Verbindung zusammenzuwirken und das Element (34) zur Deaktivierung der Rückhaltemittel in der Lage ist, mit dem proximalen Anschlag (38P) zur festen Verbindung zusammenzuwirken.

8. Spritzenanordnung nach Anspruch 6 oder 7, **dadurch gekennzeichnet, dass** Mittel zum Zurückziehen des Spritzenkörpers (12) in Richtung seiner Schutzposition eine Schubfeder (24) umfassen, welche einerseits auf einem Innenansatz der Hülse (20), genannt Rückzugansatz (32), der zwischen dem distalen Ende (20D) der Hülse (20) und dem Rückhalteansatz (28) eingesetzt ist, und andererseits auf dem Bund (22) des Spritzenkörpers (12) abgestützt ist.

9. Spritzenanordnung nach einem der vorhergehenden Ansprüche, **dadurch gekennzeichnet, dass** die Hülse (20) äußere Greifmittel (44) trägt, die dazu bestimmt sind, durch die Finger eines Benutzers ergriffen zu werden, um die Flüssigkeit durch axiale Annäherung eines proximalen Endes (16P) des Schiebers (16) und der Greifmittel (44) zu injizieren.

10. Spritzenanordnung nach Anspruch 9, **dadurch gekennzeichnet, dass** die Greifmittel einen Außenansatz (44) der Hülse (20) umfassen.

11. Hülse (20) für die Anordnung einer Flüssigkeitsinjektionsspritze nach einem der vorhergehenden Ansprüche, bei dem der Spritzenkörper (12) dazu bestimmt ist, zwischen einer aktiven Position, in der die Nadel (14) über ein distales Ende (20D) der Hülse (20) herausragt, und einer Schutzposition, in der die Nadel (14) in die Hülse (20) zurückgezogen ist, axial beweglich montiert zu sein, **dadurch gekennzeichnet, dass** sie Mittel (38D, 38P) umfasst, die dazu bestimmt sind, mit ergänzenden Mitteln (34) des Schiebers (16) zusammenzuwirken, um den Schieber (16) und die Hülse (20) in einer relativen Position dieses Schiebers (16) und dieser Hülse (20) axial fest zu verbinden, wodurch einerseits die Positionierung des Körpers (12) in seiner Schutzposition relativ zur Hülse (20) und andererseits die Positionierung des Schiebers (16) in seiner Injektionsendposition relativ zum Körper (12) ermöglicht werden.
